(19) Europäisches Patentamt
European Patent Office
Office européen des brevets

(11) EP 1 352 636 B1

(12) **FASCICULE DE BREVET EUROPEEN**

(45) Date de publication et mention de la délivrance du brevet:
**22.04.2009 Bulletin 2009/17**

(51) Int Cl.:
***A61Q 5/06*** *(2006.01)* ***A61K 8/41*** *(2006.01)*
***A61K 8/49*** *(2006.01)*

(21) Numéro de dépôt: **03291367.5**

(22) Date de dépôt: **16.07.1998**

(54) **Composition pour la teinture d'oxydation des fibres kératiniques comprenant du 2-chloro-6-méthyl-3-aminophénol et une base d'oxydation, et procédé de teinture**

2-Chlor-6-Methyl-3-Aminophenol und eine Oxidationsbase enthaltende Zusammensetzung zur oxidativen Färbung von Keratinfasern und Färbeverfahren

Oxidation dyeing composition for keratin fibers comprising 2-chloro-6-methyl-3-aminophenol and an oxidation base, and dyeing method

(84) Etats contractants désignés:
**AT BE CH DE ES FR GB IT LI NL SE**

(30) Priorité: **01.09.1997 FR 9710854**

(43) Date de publication de la demande:
**15.10.2003 Bulletin 2003/42**

(62) Numéro(s) de document de la (des) demande(s) initiale(s) en application de l'article 76 CBE:
**98939696.5 / 0 966 252**

(73) Titulaire: **L'ORÉAL**
**75008 Paris (FR)**

(72) Inventeur: **Audousset, Marie-Pascale**
**92600 Asnières (FR)**

(74) Mandataire: **Fevrier, Murielle Françoise E.**
**L'OREAL - D.I.P.I.**
**25-29 Quai Aulagnier**
**92600 Asnières (FR)**

(56) Documents cités:

| | |
|---|---|
| **EP-A- 0 591 059** | **EP-A- 0 728 465** |
| **WO-A-92/04883** | **WO-A-96/15765** |
| **WO-A-97/11674** | **WO-A-97/31886** |
| **WO-A-97/49378** | **DE-A- 3 016 008** |
| **DE-A- 4 102 907** | **DE-A- 4 122 748** |
| **DE-A- 4 205 329** | **DE-A- 4 440 955** |
| **FR-A- 2 687 399** | |

Remarques:
Le dossier contient des informations techniques présentées postérieurement au dépôt de la demande et ne figurant pas dans le présent fascicule.

EP 1 352 636 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention a pour objet une composition pour la teinture d'oxydation des fibres kératiniques, en particulier des fibres kératiniques humaines telles que les cheveux, comprenant du 2-chloro 6-méthyl 3-aminophénol à titre de coupleur, en association avec au moins une base d'oxydation convenablement sélectionnée, ainsi que le procédé de teinture mettant en oeuvre cette composition avec un agent oxydant.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols ou encore des composés hétérocycliques tels que des dérivés de pyrimidine, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec les bases d'oxydation en les associant à des coupleurs ou modificateurs de coloration convenablement choisis, ces derniers pouvant être notamment parmi des métadiamines aromatiques, des métaaminophénols, des métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, notamment dans la demande de brevet allemand DE 3 016 008 des compositions pour la teinture d'oxydation des fibres kératiniques contenant à titre de coupleur du 2-chloro 6-méthyl 3-aminophénol ou du 2-méthyl 5-chloro 3-aminophénol, en association avec des bases d'oxydation classiquement utilisées pour la teinture d'oxydation, telles que par exemple certaines paraphénylènediamines, du para-aminophénol ou encore de la tétraaminopyrimidine. De telles compositions ne sont cependant pas entièrement satisfaisantes notamment du point de vue de la tenue des colorations obtenues vis à vis des diverses agressions que peuvent subir les cheveux et en particulier vis à vis des shampooings et des déformations permanentes.

Il a également été proposé, dans les demandes de brevet WO 96/15765 et WO 96/15766, des compositions pour la teinture d'oxydation des fibres kératiniques contenant l'association spécifique du 2-chloro 6-méthyl 3-aminophénol à titre de coupleur et de bases d'oxydation particulières comme la 2-β-hydroxyéthyl paraphénylènediamine ou certains para-aminophénols particuliers comme par exemple le 3-méthyl 4-aminophénol, le 2-allyl 4-aminophénol ou bien encore le 2-aminométhyl 4-aminophénol. De telles compositions ne sont cependant pas non plus entièrement satisfaisantes notamment du point de vue de la puissance des colorations obtenues.

Il a aussi été proposé, dans la demande de brevet DE 42 05 329, une composition pour la coloration d'oxydation des cheveux comprenant une base d'oxydation choisie parmi des para-phénylènediamines de formule déterminée et un capteur, par exemple le 2-chloro 6-méthyl 3-aminophénol, et dans la demande de brevet DE 41 02 907, une composition pour la coloration d'oxydation des cheveux comprenant une base d'oxydation choisie parmi les bis-(2,5-diaminophénoxy) oxoalcanes de formule déterminée et un capteur, par exemple le 2-chloro 6-méthyl 3-aminophénol.

Or, la demanderesse vient maintenant de découvrir qu'il est possible d'obtenir de nouvelles teintures puissantes et particulièrement résistantes aux diverses agressions que peuvent subir les cheveux, en associant du 2-chloro 6-méthyl 3-aminophénol et au moins une base d'oxydation convenablement sélectionnée.

Cette découverte est à la base de la présente invention.

L'invention a donc pour premier objet une composition pour la teinture d'oxydation des fibres kératiniques et en particulier des fibres kératiniques humaines telles que les cheveux, caractérisée par le fait qu'elle comprend, dans un milieu approprié pour la teinture :

- du 2-chloro 6-méthyl 3-aminophénol et/ou au moins l'un de ses sels d'addition avec un acide, à titre de coupleur,
- et au moins une base d'oxydation choisie parmi :

  a) les pyrazolo-[1,5-a]-pyrimidines de formule (III) suivante, leurs sels d'addition avec un acide ou avec une base et leurs formes tautomères, lorsqu'il existe un équilibre tautomérique :

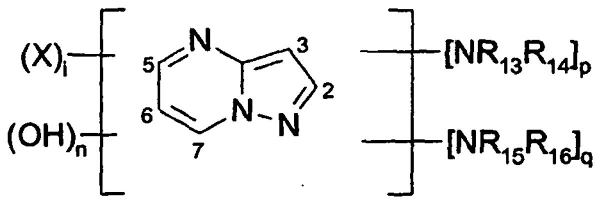

dans laquelle :

- $R_{13}$, $R_{14}$, $R_{15}$ et $R_{16}$, identique ou différents désignent, un atome d'hydrogène, un radical alkyle en $C_1$-$C_4$, un radical aryle, un radical hydroxyalkyle en $C_1$-$C_4$, un radical polyhydroxyalkyle en $C_2$-$C_4$, un radical ($C_1$-$C_4$)alcoxy alkyle en $C_1$-$C_4$, un radical aminoalkyle en $C_1$-$C_4$ (l'amine pouvant être protégée par un radical acétyle, uréido ou sulfonyle), un radical ($C_1$-$C_4$)alkylamino alkyle en $C_1$-$C_4$, un radical di-[($C_1$-$C_4$) alkyl] amino alkyle en $C_1$-$C_4$ (les radicaux dialkyles pouvant former un cycle carboné ou un hétérocycle à 5 ou 6 chaînons), un radical hydroxy($C_1$-$C_4$)alkyl- ou di-[hydroxy($C_1$-$C_4$) alkyl]-amino alkyle en $C_1$-$C_4$ ;
- les radicaux X désignent, identiques ou différents, un atome d'hydrogène, un radical alkyle en $C_1$-$C_4$, un radical aryle, un radical hydroxyalkyle en $C_1$-$C_4$, un radical polyhydroxyalkyle en $C_2$-$C_4$, un radical amino alkyle en $C_1$-$C_4$, un radical -($C_1$-$C_4$)alkyl amino alkyle en $C_1$-$C_4$, un radical di-[($C_1$-$C_4$)alkyl] amino alkyle en $C_1$-$C_4$ (les dialkyles pouvant former un cycle carboné ou un hétérocycle à 5 ou 6 chaînons), un radical hydroxy($C_1$-C4)alkyl ou di-[hydroxy($C_1$-$C_4$ )alkyl]amino alkyle en $C_1$-$C_4$, un radical amino, un radical ($C_1$-$C_4$) alkyl- ou di-[($C_1$-$C_4$)alkyl]-amino ; un atome d'halogène, un groupe acide carboxylique, un groupe acide sulfonique ;
- i vaut 0, 1, 2 ou 3 ;
- p vaut 0 ou 1 ;
- q vaut 0 ou 1 ;
- n vaut 0 ou 1 ;

sous réserve que :

- la somme p + q est différente de 0 ;
- lorsque p + q est égal à 2, alors n vaut 0 et les groupes $NR_{13}R_{14}$ et $NR_{15}R_{16}$ occupent les positions (2,3) ; (5,6) ; (6,7) ; (3,5) ou (3,7) ;
- lorsque p + q est égal à 1 alors n vaut 1 et le groupe $NR_{13}R_{14}$ (ou $NR_{15}R_{16}$) et le groupe OH occupent les positions (2,3) ; (5,6) ; (6,7) ; (3,5) ou (3,7) ;

b) les diamino-pyridines ;

ladite composition étant exempte d'un coupleur additionnel qui serait choisi parmi la 4-hydroxy indoline, la 6-hydroxy indoline et leurs sels d'addition avec un acide.

La composition de teinture d'oxydation conforme à l'invention permet d'obtenir des colorations puissantes aux nuances variées, peu sélectives et présentant d'excellentes propriétés de résistance à la fois vis à vis des agents atmosphériques tels que la lumière et les intempéries et vis à vis de la transpiration et des différents traitements que peuvent subir les cheveux (shampooings, déformations permanentes). Ces propriétés sont particulièrement remarquables notamment en ce qui concerne la résistance des colorations vis à vis des shampooings et des déformations permanentes.

Parmi les radicaux alkyle en $C_1$-$C_4$ et alcoxy en $C_1$-$C_4$ de la formule (III) ci-dessus, on peut citer notamment les radicaux méthyle, éthyle, propyle, méthyloxy et éthyloxy.

Lorsque les pyrazolo-[1,5-a]-pyrimidines de formule (III) ci-dessus sont telles qu'elles comportent un groupe hydroxyle sur l'une des positions 2, 5 ou 7 en $\alpha$ d'un atome d'azote, il existe un équilibre tautomérique représenté par exemple par le schéma suivant :

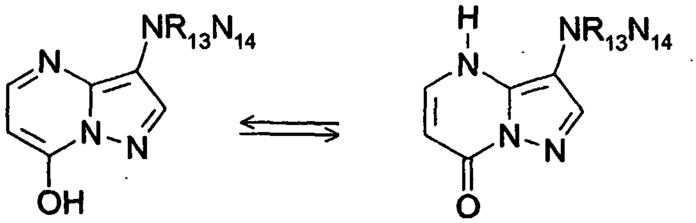

Parmi les pyrazolo-[1,5-a]-pyrimidines de formule (III), utilisables à titre de base d'oxydation dans les compositions conformes à l'invention, on peut notamment citer:

- la pyrazolo-[1,5-a]-pyrimidine-3,7-diamine ;
- la 2,5-diméthyl pyrazolo-[1,5-a]-pyrimidine-3,7-diamine ;
- la pyrazolo-[1,5-a]-pyrimidine-3,5-diamine ;
- la 2,7-diméthyl pyrazolo-[1,5-a]-pyrimidine-3,5-diamine ;
- le 3-amino pyrazolo-[1,5-a]-pyrimidin-7-ol
- le 3-amino pyrazolo-[1,5-a]-pyrimidin-5-ol
- le 2-(3-amino pyrazolo-[1,5-a]-pyrimidin-7-ylamino)-éthanol
- le 2-(7-amino pyrazolo-[1,5-a]-pyrimidin-3-ylamino)-éthanol
- le 2-[(3-Amino-pyrazolo[1,5-a]pyrimidin-7-yl)-(2-hydroxy-éthyl)-amino]-éthanol
- le 2-[(7-Amino-pyrazolo[1,5-a]pyrimidin-3-yl)-(2-hydroxy-éthyl)-amino]-éthanol
- la 5,6-diméthyl pyrazolo-[1,5-a]-pyrimidine-3,7-diamine ;
- la 2,6-diméthyl pyrazolo-[1,5-a]-pyrimidine-3,7-diamine ;
- la 2, 5, N 7, N 7-tetraméthyl pyrazolo-[1,5-a]-pyrimidine-3,7-diamine ;

et leurs sels d'addition et leurs formes tautomères, lorsqu'il existe un équilibre tautomérique.

Les pyrazolo-[1,5-a]-pyrimidines de formule (III) conformes à l'invention peuvent être préparées par cyclisation à partir d'un aminopyrazole selon les synthèses décrites dans les référencés suivantes :

- EP 628559 BEIERSDORF-LILLY
- R. Vishdu, H. Navedul, Indian J. Chem., 34b (6), 514, 1995.
- N.S. Ibrahim, K.U. Sadek, F.A. Abdel-Al, Arch. Pharm., 320, 240, 1987.
- R.H. Springer, M.B. Scholten, D.E. O'Brien, T. Novinson, J.P. Miller, R.K. Robins, J. Med. Chem., 25, 235, 1982.
- T. Novinson, R.K. Robins, T.R. Matthews, J. Med. Chem., 20, 296, 1977.
- US 3907799 ICN PHARMACEUTICALS

Les pyrazolo-[1,5-a]-pyrimidines de formule (III) conformes à l'invention peuvent également être préparées par cyclisation à partir d'hydrazine selon les synthèses décrites dans les références suivantes :

- A. McKillop et R.J. Kobilecki, Heterocycles, 6(9), 1355, 1977.
- E. Alcade, J. De Mendoza, J.M. Marcia-Marquina, C. Almera, J. Elguero, J. Heterocyclic Chem., 11(3), 423, 1974.
- K. Saito, I. Hori, M. Higarashi, H. Midorikawa, Bull. Chem. Soc. Japan, 47(2), 476, 1974.

Parmi les diamino-pyridines utilisables à titre de base d'oxydation dans les compositions tinctoriales conformes à l'invention, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Le 2-chloro 6-méthyl 3-aminophénol et/ou le ou ses sels d'addition avec un acide, représentent de préférence de 0,0001 à 5 % en poids environ du poids total de la composition tinctoriale, et encore plus préférentiellement de 0,005 à 3 % en poids environ de ce poids.

La ou les bases d'oxydation conformes à l'invention et/ou leurs sels d'addition avec un acide représentent de préférence de 0,0005 à 12 % en poids environ du poids total de la composition tinctoriale, et encore plus préférentiellement de 0,005 à 6 % en poids environ de ce poids.

Les compositions tinctoriales conformes à l'invention peuvent contenir d'autres coupleurs différents du 2-chloro

6-méthyl 3-aminophénol et/ou des colorants directs notamment pour modifier les nuances ou les enrichir en reflets.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates. Les sels d'addition avec une base utilisables dans le cadre des compositions tinctoriales de l'invention (bases d'oxydation) sont notamment ceux obtenus avec la soude, la potasse, l'ammoniaque ou les amines.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en $C_1$-$C_4$, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ et encore plus préférentiellement entre 5 et 11 environ. Il peut être ajusté à la valeur désirée au moyen d'agents acidifiants ou alcalinisants habituellement utilisés en teinture des fibres kératiniques.

Parmi les agents acidifiants on peut citer, à titre d'exemple, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (IV) suivante :

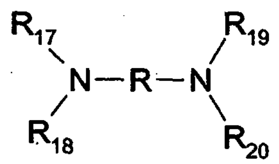

(IV)

dans laquelle R est un reste propylène éventuellement substitué par un groupement hydroxyle ou un radical alkyle en $C_1$-$C_4$ ; $R_{17}$, $R_{18}$, $R_{19}$ et $R_{20}$, identiques ou différents, représentent un atome d'hydrogène, un radical alkyle en $C_1$-$C_4$ ou hydroxyalkyle en $C_1$-$C_4$.

La composition tinctoriale selon l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à l'association conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres la composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre particulièrement préférée du procédé de teinture selon l'invention, on mélange, au moment de l'emploi, la composition tinctoriale décrite ci-dessus avec une composition oxydante contenant,

dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ,de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates, les peracides. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention.

## EXEMPLES

### EXEMPLES DE TEINTURE COMPARATIFS 1 ET 2

On a préparé les compositions tinctoriales, conformes à l'invention, suivantes (teneurs en grammes) :

| EXEMPLE | 1 | 2 (*) |
|---|---|---|
| 2-chloro 6-méthyl 3-aminophénol (Coupleur conforme à l'invention) | 0,236 | 0,236 |
| Dichlorhydrate de pyrazolo-[1,5-a]-pyrimidine-3,7-diamine (Base d'oxydation conforme à l'invention) | 0,333 | - |
| Sulfate de tétraaminopyrimidine (Base d'oxydation ne faisant pas partie de l'invention) | - | 0,357 |
| Support de teinture commun n°1 | (**) | (**) |

(suite)

| EXEMPLE | 1 | 2 (*) |
|---|---|---|
| Eau déminéralisée q.s.p. | 100 g | 100 g |

(*) : exemple ne faisant pas partie de l'invention
(**) support de teinture commun n°1 :
- Alcool oléique polyglycérolé à 2 moles de glycérol 4,0 g
- Alcool oléique polyglycérolé à 4 moles de glycérol, à 78 % de matières actives (M.A.) 5,69 g M.A.
- Acide oléique 3,0 g
- Amine oléique à 2 moles d'oxyde d'éthylène vendue sous la dénomination commerciale ETHOMEEN 012 ® par la société AKZO 7,0 g
- Laurylamino succinamate de diéthylaminopropyle, sel de sodium, à 55 % de M.A. 3,0 g M.A.
- Alcool oléique 5,0 g
- Diéthanolamide d'acide oléique 12,0 g
- Propylèneglycol 3,5 g
- Alcool éthylique 7,0 g
- Dipropylèneglycol 0,5 g
- Monométhyléther de propylèneglycol 9,0 g
- Métabisulfite de sodium en solution aqueuse, à 35 % de M.A. 0,455 g M.A.
- Acétate d'ammonium 0,8 g
- Antioxydant, séquestrant q.s.
- Parfum, conservateur q.s.
- Ammoniaque à 20 % de $NH_3$ 10 g

Il est important de noter que chaque composition tinctoriale ci-dessus contient la même quantité molaire de base d'oxydation , à savoir $1{,}5.10^{-3}$ mole.

Au moment de l'emploi, on a mélangé chaque composition tinctoriale ci-dessus avec une quantité égale en poids d'une composition oxydante constituée par une solution d'eau oxygénée à 20 volumes (6 % en poids).

Chaque composition résultante a été appliquée pendant 30 minutes sur des mèches de cheveux gris naturels à 90 % de blancs. Les mèches de cheveux ont ensuite été rincées, lavées avec un shampooing standard puis séchées.

Les mèches de cheveux ont été teintes ont ensuite été soumises à un test de résistance à l'action des shampooings.

La couleur des mèches de cheveux teintes avec les compositions 1 et 2 a été évaluée dans le système MUNSELL au moyen d'un colorimètre CM 2002 MINOLTA ®, avant le test de résistance aux shampooings.

Selon la notation MUNSELL, une couleur est définie par l'expression H V / C dans laquelle les trois paramètres désignent respectivement la teinte ou Hue (H), l'intensité ou Value (V) et la pureté ou Chromaticité (C), la barre oblique de cette expression est simplement une convention et n'indique pas un ratio.

Les mèches de cheveux teintes ont ensuite été soumises à un test de résistance à 6 shampooings (machine Ahiba-Texomat).

Pour ce faire, les mèches de cheveux ont été placées dans un panier que l'on a immergé dans une solution d'un shampooing standard à 37°C. Le panier a été soumis à un mouvement de va-et-vient vertical de fréquence variable ainsi qu'à un mouvement de rotation qui reproduisent l'action d'un frottement manuel, ce qui engendre la formation de mousse.

Après 3 minutes d'épreuve, on a retiré les mèches que l'on a rincées puis séchées.

Les mèches teintes ont été soumises à 6 épreuves de shampooing consécutives.

La couleur des mèches a été ensuite évaluée à nouveau dans le système MUNSELL au moyen d'un colorimètre CM 2002 MINOLTA ® .

La différence entre la couleur de la mèche avant les shampooings et la couleur de la mèche après les shampooings a été calculée en appliquant la formule de NICKERSON :

$$\Delta E = 0{,}4 C_0 dH + 6dV + 3dC$$

telle que décrite par exemple dans "Couleur, Industrie et Technique" ; pages 14-17 ; vol. n° 5 ; 1978.

Dans cette formule, ΔE représente la différence de couleur entre deux mèches, ΔH, ΔV et ΔC représentent la

variation en valeur absolue des paramètres H, V et C et C0 représente la pureté de la mèche par rapport à laquelle on désire évaluer la différence de couleur.

La dégradation de la couleur est d'autant plus forte que la valeur de ΔE est élevée.

Les résultats sont donnés dans le tableau ci-dessous :

| EXEMPLE | Couleur des cheveux avant les shampooings | Couleur des cheveux après les shampooings | Dégradation de la couleur | | | |
|---|---|---|---|---|---|---|
| | | | ΔH | ΔV | ΔC | ΔE |
| 1 | 5,4 R 4,2 / 5,5 | 4,8 R 4,3 / 5,0 | 0,6 | 0,1 | 0,5 | 3,4 |
| 2 (*) | 6,3 PB 4,4 / 1,5 | 1,5 GY 5,0 / 0,2 | 44,8 | 0,6 | 1,3 | 34,4 |
| (*) exemple ne faisant pas partie de l'invention | | | | | | |

Ces résultats montrent que la coloration obtenue en mettant en oeuvre la composition tinctoriale conforme à l'invention de l'exemple 1, c'est à dire contenant l'association spécifique du 2-chloro 6-méthyl 3-aminophénol et de la pyrazolo-[1,5-a]-pyrimidine-3,7-diamine, est remarquablement plus résistante que la coloration obtenue en mettant en oeuvre la composition de l'exemple 2 ne faisant pas partie de l'invention car contenant l'association du 2-chloro 6-méthyl 3-aminophénol et de la tétraaminopyrimidine qui est une base d'oxydation ne faisant pas partie de l'invention et dont l'utilisation avec le 2-chloro 6-méthyl 3-aminophénol est décrite dans la demande de brevet DE 3 016 008.

**EXEMPLES 7 et 8 DE TEINTURE**

On a préparé les compositions tinctoriales, conformes à l'invention, suivantes (teneurs en grammes) :

| EXEMPLE | 7 | 8 |
|---|---|---|
| 2-chloro 6-méthyl 3-aminophénol (coupleur) | 0,471 | 0,471 |
| Dichlorhydrate de pyrazolo-[1,5-a]-pyrimidine-3,7-diamine (base d'oxydation) | 0,666 | 0,666 |
| Support de teinture commun n°2 | (***) | - |
| Support de teinture commun n°3 | - | (****) |
| Eau déminéralisée q.s.p. | 100 g | 100 g |
| (***) support de teinture commun n°2 :<br>- Ethanol à 96 ° 18 g<br>- Métabisulfite de sodium en solution aqueuse à 35 % 0,68 g<br>- Sel pentasodique de l'acide diéthylènetriaminopentacétique 1,1 g<br>- Ammoniaque à 20 % de $NH_3$ 10 g<br>(****) support de teinture commun n°3 :<br>- Ethanol à 96 ° 18 g<br>- Métabisulfite de sodium en solution aqueuse à 35 % 0,68 g<br>- Sel pentasodique de l'acide diéthylènetriaminopentacétique 1,1 g<br>- Tampon $K_2HPO_4$ / $KH_2PO_4$ (1,5M / 1M) 10 g | | |

Au moment de l'emploi, on a mélangé chaque composition tinctoriale ci-dessus avec une quantité égale en poids d'une composition oxydante constituée par une solution d'eau oxygénée à 20 volumes (6 % en poids).

Chaque composition résultante a été appliquée pendant 30 minutes sur des mèches de cheveux gris naturels à 90 % de blancs. Les mèches de cheveux ont ensuite été rincées, lavées avec un shampooing standard puis séchées.

Les nuances obtenues figurent dans le tableau ci-dessous :

| EXEMPLE | pH de TEINTURE | NUANCE OBTENUE |
|---|---|---|
| 7 | 10 ± 0,2 | Irisé rouge puissant |
| 8 | 6,8 ± 0,2 | Rouge puissant |

## Revendications

1. Composition pour la teinture d'oxydation des fibres kératiniques humaines telles que les cheveux, **caractérisée par le fait qu'**elle comprend, dans un milieu approprié pour la teinture :

   - du 2-chloro 6-méthyl 3-aminophénol et/ou au moins l'un de ses sels d'addition avec un acide, à titre de coupleur,
   - et au moins une base d'oxydation choisie parmi:

   a) les pyrazolo-[1,5-a]-pyrimidines de formule (III) suivante, leurs sels d'addition avec un acide ou avec une base et leurs formes tautomères, lorsqu'il existe un équilibre tautomérique :

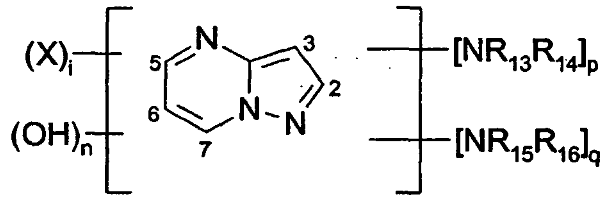

(III)

   dans laquelle :

   - $R_{13}$, $R_{14}$, $R_{15}$ et $R_{16}$, identique ou différents désignent, un atome d'hydrogène, un radical alkyle en $C_1$-$C_4$, un radical aryle, un radical hydroxyalkyle en $C_1$-$C_4$, un radical polyhydroxyalkyle en $C_2$-$C_4$, un radical ($C_1$-$C_4$)alcoxy alkyle en $C_1$-$C_4$, un radical aminoalkyle en $C_1$-$C_4$ (l'amine pouvant être protégée par un radical acétyle, uréido ou sulfonyle), un radical ($C_1$-$C_4$)alkylamino alkyle en $C_1$-$C_4$, un radical di-[($C_1$-$C_4$)alkyl] amino alkyle en $C_1$-$C_4$ (les radicaux dialkyles pouvant former un cycle carboné ou un hétérocycle à 5 ou 6 chaînons), un radical hydroxy($C_1$-$C_4$)alkyl- ou di-[hydroxy($C_1$-$C_4$) alkyl]-amino alkyle en $C_1$-$C_4$;
   - les radicaux X désignent , identiques ou différents, un atome d'hydrogène, un radical alkyle en $C_1$-$C_4$, un radical aryle, un radical hydroxyalkyle en $C_1$-$C_4$, un radical polyhydroxyalkyle en $C_2$-$C_4$, un radical amino alkyle en $C_1$-$C_4$, un radical ($C_1$-$C_4$)alkyl amino alkyle en $C_1$-$C_4$, un radical di-[($C_1$-$C_4$)alkyl] amino alkyle en $C_1$-$C_4$ (les dialkyles pouvant former un cycle carboné ou un hétérocycle à 5 ou 6 chaînons), un radical hydroxy($C_1$-C4)alkyl ou di-[hydroxy($C_1$-$C_4$ )alkyl]amino alkyle en $C_1$-$C_4$, un radical amino, un radical ($C_1$-$C_4$)alkyl- ou di-[($C_1$-$C_4$)alkyl]-amino ; un atome d'halogène, un groupe acide carboxylique, un groupe acide sulfonique ;
   - i vaut 0, 1, 2 ou 3 ;
   - p vaut 0 ou 1 ;
   - q vaut 0 ou 1 ;
   - n vaut 0 ou 1 ;

   sous réserve que :

   - la somme p + q est différente de 0 ;
   - lorsque p + q est égal à 2, alors n vaut 0 et les groupes $NR_{13}R_{14}$ et $NR_{15}R_{16}$ occupent les positions (2,3) ; (5,6) ; (6,7) ; (3,5) ou (3,7) ;
   - lorsque p + q est égal à 1 alors n vaut 1 et le groupe $NR_{13}R_{14}$ (ou $NR_{15}R_{16}$) et le groupe OH occupent les positions (2,3) ; (5,6) ; (6,7) ; (3,5) ou (3,7) ;

   b) les diamino-pyridines ;

   ladite composition étant exempte d'un coupleur additionnel qui serait choisi parmi la 4-hydroxy indoline, la 6-hydroxy indoline et leurs sels d'addition avec un acide.

2. Composition selon la revendication 1, **caractérisée par le fait que** les pyrazolo-[1,5-a]-pyrimidines de formule (III) sont choisies parmi :

   - la pyrazolo-[1,5-a]-pyrimidine-3,7-diamine ;

- la 2,5-diméthyl pyrazoto-[1,5-a]-pyrimidine-3,7-diamine ;
- la pyrazolo-[1,5-a]-pyrimidine-3,5-diamine ;
- la 2,7-diméthyl pyrazolo-[1,5-a]-pyrimidine-3,5-diamine ;
- le 3-amino pyrazolo-[1,5-a]-pyrimidin-7-ol
- le 3-amino pyrazolo-[1,5-a]-pyrimidin-5-ol
- le 2-(3-amino pyrazolo-[1,5-a]-pyrimidin-7-ylamino)-éthanol
- le 2-(7-amino pyrazolo-[1,5-a]-pyrimidin-3-ylamino)-éthanol
- le 2-[(3-Amino-pyrazolo[1,5-a]pyrimidin-7-yl)-(2-hydroxy-éthyl)amino]-éthanol
- le 2-[(7-Amino-pyrazolo[1,5-a]pyrimidin-3-yl)-(2-hydroxy-éthyl)-amino]-éthanol
- la 5,6-diméthyl pyrazolo-[1,5-a]-pyrimidine-3,7-diamine ;
- la 2,6-diméthyl pyrazolo-[1,5-a]-pyrimidine-3,7-diamine ;
- la 2, 5, N 7, N 7-tetraméthyl pyrazolo-[1,5-a]-pyrimidine-3,7-diamine ;

et leurs sels d'addition et leurs formes tautomères, lorsqu'il existe un équilibre tautomérique.

**3.** Composition selon la revendication 1 ou 2, **caractérisée par le fait que** les diamino-pyridines sont choisies parmi la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

**4.** Composition selon l'une quelconque des revendications précédentes, **caractérisée par le fait que** le 2-chloro 6-méthyl 3-aminophénol et/ou le ou ses sels d'addition avec un acide représente de 0,0001 à 5% en poids du poids total de la composition tinctoriale.

**5.** Composition selon la revendication 4, **caractérisée par le fait que** le 2-chloro 6-méthyl 3-aminophénol et/ou le ou ses sels d'addition avec un acide représente de 0,005 à 3% en poids du poids total de la composition tinctoriale.

**6.** Composition selon l'une quelconque des revendications précédentes, **caractérisée par le fait que** la ou les bases d'oxydation représentent de 0,0005 à 12% en poids du poids total de la composition tinctoriale.

**7.** Composition selon la revendication 6, **caractérisée par le fait que** la ou les bases d'oxydation représentent de 0,005 à 6% en poids du poids total de la composition tinctoriale.

**8.** Composition selon l'une quelconque des revendications précédentes, **caractérisée par le fait que** les sels d'addition avec un acide sont choisis parmi les chlorhydrates, les bromhydrates, les sulfates et les tartrates, les lactates et les acétates et que les sels d'addition avec une base sont ceux obtenus avec la soude, la potasse, l'ammoniaque ou les amines.

**9.** Composition selon l'une quelconque des revendications précédentes, **caractérisée par le fait que** le milieu approprié pour la teinture (ou support) est constitué par de l'eau ou par un mélange d'eau et d'au moins un solvant organique choisi parmi les alcanols inférieurs en $C_1$-$C_4$, le glycérol, les glycols et éthers de glycols, les alcools aromatiques, les produits analogues et leurs mélanges.

**10.** Composition selon l'une quelconque des revendications précédentes, **caractérisée par le fait qu'**elle présente un pH compris entre 3 et12.

**11.** Composition selon l'une quelconque des revendications précédentes, **caractérisée par le fait qu'**elle se présente sous forme de liquides, de crèmes, de gels, ou sous toute autre forme appropriée pour réaliser une teinture des fibres kératiniques, et notamment des cheveux humains.

**12.** Procédé de teinture des fibres kératiniques et en particulier des fibres kératiniques humaines telles que les cheveux **caractérisé par le fait que** l'on applique sur ces fibres au moins une composition tinctoriale telle que définie à l'une quelconque des revendications 1 à 11, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

**13.** Procédé selon la revendication 12, **caractérisé par le fait que** l'agent oxydant présent dans la composition oxydante est choisi parmi le peroxyde d'hydrogène, le peroxyde d'urée, les bromates de métaux alcalins, les persels tels que les perborates et persulfates, les peracides.

**14.** Dispositif à plusieurs compartiments, ou "kit" de teinture à plusieurs compartiments, dont un premier compartiment renferme une composition tinctoriale telle que définie à l'une quelconque des revendications 1 à 11 et un second compartiment renferme une composition oxydante.

## Claims

**1.** Composition for the oxidation dyeing of human keratinous fibres, such as hair, **characterized in that** it comprises, in a medium appropriate for dyeing:

- 2-chloro-6-methyl-3-aminophenol and/or at least one of its addition salts with an acid, as coupler, and
- at least one oxidation base chosen from:

a) pyrazolo[1,5-a]pyrimidines of following formula (III), their addition salts with an acid or with a base and their tautomeric forms, when a tautomeric equilibrium exists:

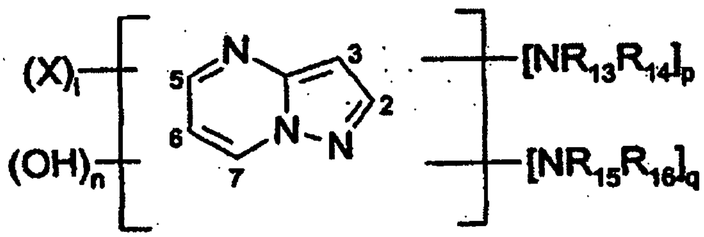

(III)

in which:

- $R_{13}$, $R_{14}$, $R_{15}$ and $R_{16}$, which are identical or different, denote a hydrogen atom, a $C_1$-$C_4$ alkyl radical, an aryl radical, a $C_1$-$C_4$ hydroxyalkyl radical, a $C_2$-$C_4$ polyhydroxyalkyl radical, a ($C_1$-$C_4$) alkoxy($C_1$-$C_4$) alkyl radical, a $C_1$-$C_4$ aminoalkyl radical (it being possible for the amine to be protected by an acetyl, ureido or sulphonyl radical), a ($C_1$-$C_4$) alkylamino ($C_1$-$C_4$) alkyl radical, a di[($C_1$-$C_4$)alkyl]amino($C_1$-$C_4$) alkyl radical (it being possible for the dialkyl radicals to form a carbon ring or a heterocycle with 5 or 6 ring members) or a hydroxy($C_1$-$C_4$)alkyl- or di(hydroxy($C_1$-$C_4$)alkyl]amino($C_1$-$C_4$) alkyl radical;
- the X radicals, which are identical or different, denote a hydrogen atom, a $C_1$-$C_4$ alkyl radical, an aryl radical, a $C_1$-$C_4$ hydroxyalkyl radical, a $C_2$-$C_4$ polyhydroxyalkyl radical, a $C_1$-$C_4$ aminoalkyl radical, a ($C_1$-$C_4$)alkylamino($C_1$-$C_4$)alkyl radical, a di [($C_1$-$C_4$)alkyl] amino ($C_1$-$C_4$)alkyl radical (it being possible for the dialkyls to form a carbon ring or a heterocycle with 5 or 6 ring members), a hydroxy($C_1$-$C_4$) alkyl- or di[hydroxy($C_1$-$C_4$)alkyl]-amino($C_1$-$C_4$)alkyl radical, an amino radical, a ($C_1$-$C_4$)alkyl- or di[($C_1$-$C_4$) alkyl]amino radical, a halogen atom, a carboxylic acid group or a sulphonic acid group;
- i has the value 0, 1, 2 or 3;
- p has the value 0 or 1;
- q has the value 0 or 1;
- n has the value 0 or 1;

with the proviso that:

- the sum p + q is other than 0;
- when p + q is equal to 2, then n has the value 0 and the $NR_{13}R_{14}$ and $NR_{15}R_{16}$ groups occupy the (2,3), (5,6), (6,7), (3,5) or (3,7) positions;
- when p + q is equal to 1, then n has the value 1 and the $NR_{13}R_{14}$ (or $NR_{15}R_{16}$) group and the OH group occupy the (2,3), (5,6), (6,7), (3,5) or (3,7) positions;

b) diaminopyridines;

the said composition being devoid of an additional coupler which would be chosen from 4-hydroxyindoline, 6-hydroxyindoline and their addition salts with an acid.

2. Composition according to Claim 1, **characterized in that** the pyrazolo[1,5-a]pyrimidines of formula (III) are chosen from:

- pyrazolo[1,5-a]pyrimidine-3,7-diamine;
- 2,5-dimethylpyrazolo[1,5-a]pyrimidine-3,7-diamine;
- pyrazolo[1,5-a]pyrimidine-3,5-diamine;
- 2,7-dimethylpyrazolo[1,5-a]pyrimidine-3,5-diamine;
- 3-aminopyrazolo[1,5-a]pyrimidin-7-ol;
- 3-aminopyrazolo[1,5-a]pyrimidin-5-ol;
- 2-(3-aminopyrazolo[1,5-a]pyrimidin-7-ylamino)ethanol;
- 2-(7-aminopyrazolo[1,5-a]pyrimidin-3-ylamino)ethanol;
- 2-[(3-aminopyrazolo[1,5-a]pyrimidin-7-yl)(2-hydroxyethyl)amino]ethanol;
- 2-[(7-aminopyrazolo[1,5-a]pyrimidin-3-yl)(2-hydroxyethyl)amino]ethanol;
- 5,6-dimethylpyrazolo[1,5-a]pyrimidine-3,7-diamine;
- 2,6-dimethylpyrazolo[1,5-a]pyrimidine-3,7-diamine;
- 2,5,N7,N7-tetramethylpyrazolo[1,5-a]pyrimidine-3,7-diamine;

and their addition salts and their tautomeric forms, when there exists a tautomeric equilibrium.

3. Composition according to Claim 1 or 2, **characterized in that** the diaminopyridines are chosen from 2,5-diaminopyridine, 2-(4-methoxyphenyl)amino-3-aminopyridine, 2,3-diamino-6-methoxypyridine, 2-(β-methoxyethyl)amino-3-amino-6-methoxypyridine, 3,4-diaminopyridine, and their addition salts with an acid.

4. Composition according to any one of the preceding claims, **characterized in that** 2-chloro-6-methyl-3-aminophenol and/or its addition salt or salts with an acid represent from 0.0001 to 5% by weight of the total weight of the dyeing composition.

5. Composition according to Claim 4, **characterized in that** 2-chloro-6-methyl-3-aminophenol and/or its addition salt or salts with an acid represent from 0.005 to 3% by weight of the total weight of the dyeing composition.

6. Composition according to any one of the preceding claims, **characterized in that** the oxidation base or bases represent from 0.0005 to 12% by weight of the total weight of the dyeing composition.

7. Composition according to Claim 6, **characterized in that** the oxidation base or bases represent from 0.005 to 6% by weight of the total weight of the dyeing composition.

8. Composition according to any one of the preceding claims, **characterized in that** the addition salts with an acid are chosen from hydrochlorides, hydrobromides, sulphates and tartrates, lactates and acetates and **in that** the addition salts with a base are those obtained with sodium hydroxide, potassium hydroxide, ammonia or amines.

9. Composition according to any one of the preceding claims, **characterized in that** the medium appropriate for dyeing (or vehicle) is composed of water or of a mixture of water and at least one organic solvent chosen from lower $C_1$-$C_4$ alkanols, glycerol, glycols and glycol ethers, aromatic alcohols, analogous products and their mixtures.

10. Composition according to any one of the preceding claims, **characterized in that** it exhibits a pH of between 3 and 12.

11. Composition according to any one of the preceding claims, **characterized in that** it is provided in the form of liquids, creams or gels, or in any other form appropriate for carrying out dyeing of keratinous fibres and in particular of human hair.

12. Process for dyeing keratinous fibres and in particular human keratinous fibres, such as hair, **characterized in that** at least one dyeing composition as defined in any one of Claims 1 to 11 is applied to these fibres and **in that** the colour is developed at acidic, neutral or alkaline pH using an oxidizing agent which is added only at the time of use to the dyeing composition or which is present in an oxidizing composition applied simultaneously or sequentially in a separate fashion.

13. Process according to Claim 12, **characterized in that** the oxidizing agent present in the oxidizing composition is chosen from hydrogen peroxide, urea hydrogen peroxide, alkali metal bromates, persalts, such as perborates and

persulphates, or peracids.

**14.** Dyeing multi-compartment device or kit with several compartments, a first compartment of which includes a dyeing composition as defined in any one of Claims 1 to 11 and a second compartment of which includes an oxidizing composition.

## Patentansprüche

**1.** Zusammensetzung zum oxidativen Färben von menschlichen Keratinfasern, wie Haaren, **dadurch gekennzeichnet, dass** sie in einem zum Färben geeigneten Medium enthält:

- das 2-Chlor-6-methyl-3-aminophenol und/oder mindestens eines seiner Additionssalze mit einer Säure als Kuppler,
- mindestens eine Oxidationsbase, die ausgewählt ist unter:

a) den Pyrazolo-[1,5-a]-pyrimidinen der folgenden Formel (III), den Additionssalzen dieser Verbindungen mit einer Säure oder mit einer Base und ihren tautomeren Formen, falls ein tautomeres Gleichgewicht existiert:

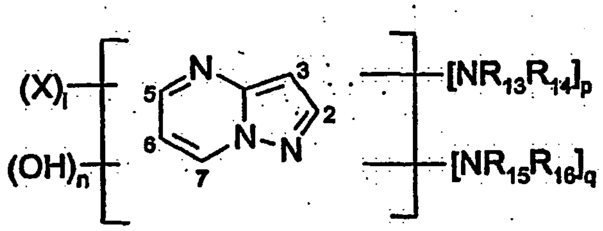

(III)

worin bedeuten:

- $R_{13}$, $R_{14}$, $R_{15}$ und $R_{16}$, die identisch oder voneinander verschieden sind, ein Wasserstoffatom, $C_{1-4}$-Alkyl, Aryl, $C_{1-4}$-Hydroxyalkyl, $C_{2-4}$-Polyhydroxyalkyl, $C_{1-4}$-Alkoxy-$C_{1-4}$-alkyl, $C_{1-4}$-Aminoalkyl (wobei die Aminogruppe mit Acetyl, Ureido oder Sulfonyl geschützt sein kann), $C_{1-4}$-Alkylamino-$C_{1-4}$-alkyl, Di-[$C_{1-4}$-alkyl]amino-$C_{1-4}$-alkyl (wobei die beiden Alkylgruppen einen Kohlenstoffring oder Heterocyclus mit 5 oder 6 Gliedern bilden können), Hydroxy-$C_{1-4}$-alkylamino-$C_{1-4}$-alkyl oder Di-[hydroxy-$C_{1-4}$-alkyl] amino-$C_{1-4}$-alkyl;
- die Gruppen X, die identisch oder voneinander verschieden sind, ein Wasserstoffatom, $C_{1-4}$-Alkyl, Aryl, $C_{1-4}$-Hydroxyalkyl, $C_{2-4}$-Polyhydroxyalkyl, $C_{1-4}$-Aminoalkyl, $C_{1-4}$-Alkylamino-$C_{1-4}$-alkyl, Di-[$C_{1-4}$-alkyl]amino-$C_{1-4}$-alkyl (wobei die beiden Alkylgruppen einen Kohlenstoffring oder Heterocyclus mit 5 oder 6 Gliedern bilden können), Hydroxy-$C_{1-4}$-alkylamino-$C_{1-4}$-alkyl, Di-[hydroxy-$C_{1-4}$-alkyl]amino-$C_{1-4}$-alkyl, Amino, $C_{1-4}$-Alkylamino, Di-[$C_{1-4}$-alkyl] amino, Halogen, Carboxy oder eine Sulfonsäuregruppe;
- i 0, 1, 2 oder 3;
- p 0 oder 1:
- q 0 oder 1;
- n 0 oder 1;

mit der Maßgabe, dass
die Summe p + q von Null verschieden ist;
n Null bedeutet und die Gruppen $NR_{13}R_{14}$ und $NR_{15}R_{16}$ die Stellungen (2,3); (5,6); (6,7); (3,5) oder (3,7) einnehmen, wenn p + q 2 ist;
n 1 bedeutet und die Gruppe $NR_{13}R_{14}$ (oder $NR_{15}R_{16}$) und die OH-Gruppe die Stellungen (2,3); (5,6); (6,7); (3,5) oder (3,7) einnehmen, wenn p + q 1 ist;
b) Diamino-pyridinen;

wobei die Zusammensetzung keinen ergänzenden Kuppler enthält, der unter 4-Hydroxy-indolin, 6-Hydroxy-indolin

und deren Additionssalzen mit einer Säure ausgewählt ist.

2. Zusammensetzung nach Anspruch 1, **dadurch gekennzeichnet, dass** die Pyrazolo-[1,5-a]-pyrimidine der Formel (III) ausgewählt sind unter:

- Pyrazolo-[1,5-a]-pyrimidin-3,7-diamin,
- 2,5-Dimethyl-pyrazolo-[1,5-a]-pyrimidin-3,7-diamin,
- Pyrazolo-[1,5-a]-pyrimidin-3,5-diamin,
- 2,7-Dimethyl-pyrazolo-[1,5-a]-pyrimidin-3,5-diamin,
- 3-Amino-pyrazolo-[1,5-a]-pyrimidin-7-ol,
- 3-Amino-pyrazolo-[1,5-a]-pyrimidin-5-ol,
- 2-(3-Amino-pyrazolo-[1,5-a]-pyrimidin-7-ylamino)-ethanol,
- 2-(7-Amino-pyrazolo-[1,5-a]-pyrimidin-3-ylamino)-ethanol,
- 2-[(3-Amino-pyrazolo-[1,5-a]-pyrimidin-7-yl)-(2-hydroxyethyl)-amino]-ethanol,
- 2-[(7-Amino-pyrazolo-[1,5-a]-pyrimidin-3-yl)-(2-hydroxyethyl)-amino]-ethanol,
- 5,6-Dimethyl-pyrazolo-[1,5-a]-pyrimidin-3,7-diamin,
- 2,6-Dimethyl-pyrazolo-[1,5-a]-pyrimidin-3,7-diamin,
- 2,5,N7,N7-Tetramethyl-pyrazolo-[1,5-a]-pyrimidin-3,7-diamin,
- und deren Additionssalzen und ihren tautomeren Formen, falls ein tautomeres Gleichgewicht existiert.

3. Zusammensetzung nach Anspruch 1 oder 2, **dadurch gekennzeichnet, dass** die Diamino-pyridine unter 2,5-Diamino-pyridin, 2-(4-Methoxyphenyl)amino-3-aminopyridin, 2,3-Diamino-6-methoxypyridin, 2-(β-Methoxyethyl) amino-3-amino-6-methoxypyridin, 3,4-Diaminopyridin und ihren Additionssalzen mit einer Säure ausgewählt sind.

4. Zusammensetzung nach einem der vorhergehenden Ansprüche, **dadurch gekennzeichnet, dass** das 2-Chlor-6-methyl-3-aminophenol und/oder das oder die Additionssalz(e) dieser Verbindung mit einer Säure 0,0001 bis 5 Gew.-% des Gesamtgewichts der Farbmittelzusammensetzung ausmachen.

5. Zusammensetzung nach Anspruch 4, **dadurch gekennzeichnet, dass** das 2-Chlor-6-methyl-3-aminophenol und/oder das oder die Additionssalz(e) dieser Verbindung mit einer Säure 0,005 bis 3 Gew.-% des Gesamtgewichts der Farbmittelzusammensetzung ausmachen.

6. Zusammensetzung nach einem der vorhergehenden Ansprüche, **dadurch gekennzeichnet, dass** die Oxidationsbase(n) 0,0005 bis 12 Gew.-% des Gesamtgewichts der Farbmittelzusammensetzung ausmachen.

7. Zusammensetzung nach Anspruch 6, **dadurch gekennzeichnet, dass** die Oxidationsbase(n) 0,005 bis 6 Gew.-% des Gesamtgewichts der Farbmittelzusammensetzung ausmachen.

8. Zusammensetzung nach einem der vorhergehenden Ansprüche, **dadurch gekennzeichnet, dass** die Additionssalze mit einer Säure unter den Hydrochloriden, Hydrobromiden, Sulfaten, Tartraten, Lactaten und Acetaten und die Additionssalze mit einer Base unter den Salzen ausgewählt sind, die mit Natriumhydroxid, Kaliumhydroxid, Ammoniak oder Aminen erhalten werden.

9. Zusammensetzung nach einem der vorhergehenden Ansprüche, **dadurch gekennzeichnet, dass** das das zum Färben geeignete Medium (oder der Träger) aus Wasser oder einem Gemisch von Wasser und mindestens einem organischen Lösungsmittel besteht, das unter den niederen $C_{1-4}$-Alkanolen, Glycerin, Glycolen und Glycolethern, aromatischen Alkoholen, analogen Produkten und deren Gemischen ausgewählt ist.

10. Zusammensetzung nach einem der vorhergehenden Ansprüche, **dadurch gekennzeichnet, dass** sie einen pH-Wert im Bereich von 3 bis 12 besitzt.

11. Zusammensetzung nach einem der vorhergehenden Ansprüche, **dadurch gekennzeichnet, dass** sie als Flüssigkeit, Creme, Gel oder in beliebigen anderen Formen, die geeignet sind, Keratinfasern und insbesondere menschliche Haare zu färben, ausgewählt sind.

12. Verfahren zum oxidativen Färben von Keratinfasern und insbesondere menschlichen Keratinfasern, wie Haaren, **dadurch gekennzeichnet, dass** auf die Fasern mindestens eine Farbmittelzusammensetzung nach einem der Ansprüche 1 bis 11 aufgebracht wird und die Farbe bei einem sauren, neutralen oder alkalischen pH-Wert mit einem

Oxidationsmittel gebildet wird, das bei der Anwendung zu der Farbmittelzusammensetzung gegeben wird oder das in einer oxidierenden Zusammensetzung vorliegt, die gleichzeitig oder getrennt davon anschließend aufgetragen wird.

**13.** Verfahren nach Anspruch 12, **dadurch gekennzeichnet, dass** das in der oxidierenden Zusammensetzung enthaltene Oxidationsmittel unter Wasserstoffperoxid, Harnstoffperoxid, Alkalimetallbromaten, Salzen von Persäuren, wie Perboraten und Persulfaten, und Persäuren ausgewählt ist.

**14.** Vorrichtung mit mehreren Abteilungen oder "Kit" zum Färben mit mehreren Abteilungen, wobei eine erste Abteilung eine Farbmittelzusammensetzung nach einem der Ansprüche 1 bis 11 und eine zweite Abteilung eine oxidierende Zusammensetzung enthält.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- DE 3016008
- WO 9615765 A
- WO 9615766 A
- DE 4205329
- DE 4102907
- EP 628559 A
- US 3907799 A
- GB 1026978 A
- GB 1153196 A
- FR 2586913


### Littérature non-brevet citée dans la description


- **R. VISHDU ; H. NAVEDUL.** *Indian J. Chem.,* 1995, vol. 34b (6), 514
- **N.S. IBRAHIM ; K.U. SADEK ; F.A. ABDEL-AL.** *Arch. Pharm.,* 1987, vol. 320, 240
- **R.H. SPRINGER ; M.B. SCHOLTEN ; D.E. O'BRIEN ; T. NOVINSON ; J.P. MILLER ; R.K. ROBINS.** *J. Med. Chem.,* 1982, vol. 25, 235
- **T. NOVINSON ; R.K. ROBINS ; T.R. MATTHEWS.** *J. Med. Chem.,* 1977, vol. 20, 296
- **A. MCKILLOP ; R.J. KOBILECKI.** *Heterocycles,* 1977, vol. 6 (9), 1355
- **E. ALCADE ; J. DE MENDOZA ; J.M. MARCIA-MARQUINA ; C. ALMERA ; J. ELGUERO.** *J. Heterocyclic Chem.,* 1974, vol. 11 (3), 423
- **K. SAITO ; I. HORI ; M. HIGARASHI ; H. MIDORIKAWA.** *Bull. Chem. Soc. Japan,* 1974, vol. 47 (2), 476
- *Couleur, Industrie et Technique,* 1978, vol. 5, 14-17